# EUROPÄISCHE PATENTANMELDUNG

(11) **EP 2 524 906 A1**
(43) Veröffentlichungstag der Anmeldung: **21.11.2012**
(21) Anmeldenummer: 11166843.0
(22) Anmeldetag: 20.05.2011
(51) Int. Cl.: C07C 37/84, C07C 39/06

(54) **Verfahren zur Herstellung von para-Thymol**

(71) Anmelder: LANXESS Deutschland GmbH, 51369 Leverkusen (DE)
(72) Erfinder: Heuer, Lutz, Dr., 41542 Dormagen (DE); Herzhoff, Michael, Dr., 53804 Much (DE); Böger, Uwe, Dr., 51375 Leverkusen (DE)

(57) **Zusammenfassung**

Die vorliegende Erfindung betrifft ein Verfahren zur Herstellung von 4-Isopropyl-3-methyl-phenol (p-Thymol) aus Destillationsrückständen der Thymolherstellung.

## Beschreibung

Die vorliegende Erfindung betrifft ein Verfahren zur Herstellung von 4-Isopropyl-3-methyl-phenol (p-Thymol) aus Destillationsrückständen der Thymolherstellung.

4-Isopropyl-3-methyl-phenol wird beispielsweise als antibakterielles und mikrobizides Mittel in Kosmetika und Mundwässern mit Anti-Plaque-Wirkung sowie Fuss- und Haarpflegemitteln mit guter Hautverträglichkeit eingesetzt. Die Herstellung von 4-Isopropyl-3-methyl-phenol ist prinzipiell bekannt.

So beschreibt US 3,331,879 die Umsetzung von meta-Kresol (m-Kresol, 3-Methylphenol) mit Propen an einem Zirkoniumkatalysator, wobei im Wesentlichen Thymol (2-Isopropyl-5-methylphenol), aber auch viele aromatische Nebenprodukte entstehen. Als ein Nebenprodukt wurde 4-Isopropyl-3-methyl-phenol mit einem Gehalt von 2 % in der Reaktionsmischung bzw. 4,4 % nach einer ersten Destillation identifiziert. Die Isolierung des 4-Isopropyl-3-methyl-phenols wird nicht beschrieben.

DE 2139622 OS beschreibt den Anfall von bis zu 19,5 % 4-Isopropyl-3-methyl-phenol bei der Umsetzung von m-Kresol mit Propen an einem sauren Zinkkatalysator. Auch hier wird die Isolierung des 4-Isopropyl-3-methyl-phenols nicht beschrieben.

DE 2528303 OS beschreibt den Anfall von ca. 2 % 4-Isopropyl-3-methyl-phenol bei der Umsetzung von meta-Kresol mit Propen an einem basischen Aluminiumoxid-Katalysator. Isolierung des 4-Isopropyl-3-methyl-phenols aus den vielfältigen Nebenprodukten wird nicht beschrieben.

Weiterhin ist aus US 2,603,662 bekannt 4-Isopropyl-3-methyl-phenol über einen aufwändigen Prozess als Nebenprodukt bei der Umsetzung von meta-Kresol mit Isobuten zu gewinnen.

Allen vorgenannten Verfahren ist gemeinsam, dass 4-Isopropyl-3-methyl-phenol als Nebenkomponente bei der Alkylierung von m-Kresol mit so vielen anderen Nebenkomponenten anfällt, dass eine Gewinnung entweder nicht vorgenommen wurde oder außerordentlich umständlich erfolgt.

Aus DE 102007035515 A ist ein Verfahren bekannt , bei dem Thymol und nicht-umgesetztes meta-Kresol zunächst destillativ aus einer Reaktionsmischung aus der Thymolherstellung weitgehend abgetrennt werden und der dabei verbleibende Rückstand destilliert wird, um schwerst- oder nichtflüchtige Substanzen abzutrennen, und das so erhaltene Destillat nach Zusatz von bis zu 5 Gew.-% Wasser kristallisiert wird oder der verbleibende Rückstand nach Zusatz von bis zu 5 Gew.-% Wasser kristallisiert wird und der kristallisierte Rückstand durch Destillation von schwerst- oder nichtflüchtigen Substanzen getrennt wird.

Die erhaltene Reinheit des 4-Isopropyl-3-methyl-phenols betrug 95,1 %.

Es bestand daher das Bedürfnis, ein Verfahren bereitzustellen, mit dem 4-Isopropyl-3-methyl-phenol in effizienter Weise und hoher Reinheit gewonnen werden kann.

Es wurde nun ein Verfahren zur Herstellung von 4-Isopropyl-3-methyl-phenol aus Reaktionsmischungen gefunden, die bei der Umsetzung von meta-Kresol mit Propen in Gegenwart eines Katalysators erhalten werden, das dadurch gekennzeichnet ist, dass
a) Thymol und nicht-umgesetztes meta-Kresol destillativ aus der Reaktionsmischung weitgehend abgetrennt werden wobei ein Destillationsrückstand gewonnen wird und
b) aus dem gemäß Schritt a) verbleibenden Destillationsrückstand rohes 4-Isopropyl-3-methylphenol durch Kristallisation gewonnen wird und
c) das gemäß Schritt b) erhaltene, rohe 4-Isopropyl-3-methyl-phenol mit einem organischen Lösungsmittel behandelt wird, das zu mindestens 80 Gew.-% aus Kohlenwasserstoffen oder halogenierten Kohlenwasserstoffen oder einer Mischung daraus besteht.

Der Rahmen der Erfindung umfasst neben den genannten Bereichen und Vorzugsbereichen von Formeln und Parametern auch beliebige Kombinationen davon, selbst wenn sie aus praktischen Gründen nachstehend nicht vollständig explizit aufgeführt sind.

Bei der Alkylierung von meta-Kresol mit Propen in Gegenwart eines Katalysators, die in einer dem Fachmann bekannten Art und Weise durchgeführt werden kann (siehe z.B. DE 3824284 OS oder DE 2528303 OS) entsteht typischerweise ein Reaktionsgemisch, das neben einer Hauptmenge an Thymol auch etwa 1 bis 3 Gew.-% 4-Isopropyl-3-methylphenol enthält.

In einem Schritt a) des erfindungsgemäßen Verfahrens werden Thymol und nicht-umgesetztes meta-Kresol aus der Reaktionsmischung destillativ weitgehend abgetrennt. Der Begriff weitgehend bedeutet dabei, dass der verbleibende Rückstand einen Anteil an Thymol und meta-Kresol von zusammengenommen insgesamt 80 % oder weniger, bevorzugt 55 % oder weniger und besonders bevorzugt 30 % oder weniger aufweist.

Die Destillation kann dabei in an sich bekannter Weise beispielsweise diskontinuierlich oder kontinuierlich durchgeführt werden, wobei eine kontinuierliche Destillation unter gegenüber Normaldruck vermindertem Druck bevorzugt ist, der beispielsweise 1 bis 950 hPa, bevorzugt von 50 bis 950 und besonders bevorzugt von 50 bis 150 hPa betragen kann.

Die Temperatur bei der Destillation beträgt am Kolonnenkopf beispielsweise von 100 bis 225°C und bevorzugt von 140 bis 155°C, die Sumpftemperatur beträgt beispielsweise von 120 bis 260°C und bevorzugt von 170 bis 190°C wobei dem Fachmann klar ist, dass die Temperaturen bei der Destillation am Kolonnenkopf und im Sumpf miteinander sowie mit dem Destillationsdruck korrelieren. Geeignete Destillationsbedingungen sind für den Fachmann ohne Weiteres ermittelbar.

Vorzugsweise erfolgt die Destillation mit Hilfe eines Kurzwegverdampfers, einer Kolonne ohne Einbauten oder durch einen Fallfilmverdampfer oder auch einen Dünnschichtverdampfer. Für die Destillation ist eine theoretische Trennstufe ausreichend. Die Anwendung mehr als einer Trennstufe ist natürlich möglich aber unnötig.

Typischerweise enthält der verbleibende Rückstand neben 4-Isopropyl-3-methylphenol 20 bis 30 weiteren Nebenkomponenten niedermolekularer Struktur sowie polymere Nebenkomponenten. Nach der Destillation beträgt der Gehalt von 4-Isopropyl-3-methylphenol im verbleibenden, üblicherweise schwarz gefärbten Rückstand, typischerweise 10 bis 30 Gew.-%.

Gemäß Schritt b) wird aus dem gemäß Schritt a) verbleibenden Rückstand rohes 4-Isopropyl-3-methyl-phenol kristallisiert. Vorzugsweise gelingt dies durch Abkühlen des gemäß Schritt a) verbleibenden Rückstandes, wobei die Temperaturdifferenz beim Abkühlen beispielsweise 30 K oder mehr, vorzugsweise 40 K oder mehr, bevorzugt 40 bis 100 K betragen kann.

Da der gemäß Schritt a) verbleibende Rückstand aus der Destillation nach Entnahme aus der Destillationsvorrichtung typischerweise eine Temperatur von 140 bis 180°C und bevorzugt von 120 bis 140°C aufweist, gelingt die Kristallisation besonders gut durch einfaches Abkühlen des Rückstandes zum Beispiel auf Temperaturen von 30°C oder weniger bevorzugt auf 25°C oder weniger, besonders bevorzugt auf zwischen -10° und 25°C.

Während des Abkühlens kann gerührt werden oder nicht wobei Rühren bevorzugt ist.

In einer Ausführungsform des erfindungsgemäßen Verfahrens kann die Kristallisation durch Animpfen mit kristallinem 4-Isopropyl-3-methyl-phenols induziert oder beschleunigt werden.

Die Zeitdauer der Kristallisation beträgt typischerweise 0,5 bis 200 Stunden vorzugsweise 1 bis 48 stunden und besonders bevorzugt 2 bis 24 Stunden.

Gemäß Schritt b) wird durch Kristallisation rohes 4-Isopropyl-3-methyl-phenol erhalten. Der Reinheitsgrad des so erhaltenen rohen 4-Isopropyl-3-methyl-phenols beträgt etwa 75 bis 90 Gew.-%, vorzugsweise 80 bis 90 Gew.-%.

Die Abtrennung des rohen 4-Isopropyl-3-methyl-phenols von der Mutterlauge kann in an sich bekannter Weise durch Sedimentation und Dekantieren, Filtration oder Zentrifugation oder andere, dem Fachmann bekannte fest-flüssig Trennoperationen erfolgen.

Der Anteil des 4-Isopropyl-3-methyl-phenols beträgt etwa 60 bis 90 Gew.-%, vorzugsweise 70 bis 90 Gew.-% bezogen auf den Gehalt an 4-Isopropyl-3-methyl-phenol in dem für Schritt b) eingesetzten Destillationsrückstand.

Das gemäß Schritt b) gewonnene, rohe 4-Isopropyl-3-methylphenol wird in Schritt c) mit einem organischen Lösungsmittel behandelt, das zu mindestens 80 Gew.-% aus Kohlenwasserstoffen oder halogenierten Kohlenwasserstoffen oder einer Mischung daraus besteht. Unter dem Begriff "Behandlung" wird im Rahmen der Erfindung sowohl "Waschen mit" als auch "Umkristallisieren aus" sowie beliebige Kombinationen dieser Prozesse verstanden.

Das für die Behandlung benutzte organische Lösungsmittel besteht zu mindestens 80 Gew.-%, vorzugsweise zu mindestens 90 Gew.-% und besonders bevorzugt zu mindestens 98 Gew.-% aus Kohlenwasserstoffen oder halogenierten Kohlenwasserstoffen oder einer Mischung daraus, wobei besonders bevorzugt als Lösungsmittel ausschließlich Kohlenwasserstoffe oder halogenierte Kohlenwasserstoffe verwendet werden, wie sie in technischer oder Laborqualität kommerziell erhältlich sind.

Kohlenwasserstoffe oder halogenierte Kohlenwasserstoffe sind bevorzugt solche, die bei 1013 hPa einen Siedepunkt von 30 bis 160°C aufweisen.

Bevorzugte Kohlenwasserstoffe sind Cyclopentan, Cyclohexan, n-Hexan, 2-Methylpentan, 3-Methylpentan, Methylcyclopentan, 2,2-Dimethylbutan, 2,3-Dimethylbutan, Methylcyclohexan, 2,2-Dimethylpentan, 2,3-Dimethylpentan, 2,4-Dimethylpentan, n-Heptan, n-Oktan, Isooctan oder Mischungen davon, wobei Methylcyclohexan besonders bevorzugt ist.

Bevorzugte halogenierte Kohlenwasserstoffe sind Dichlormethan, Chlorbenzol, die isomeren Dichlorbenzole oder Mischungen der vorgenannten Lösungsmittel, wobei Dichlormethan und Chlorbenzol bevorzugt sind.

Sofern gewaschen wird, kann das Waschen beispielsweise mit 0,5 bis 100 Gewichtsäquivalenten des Lösungsmittels bezogen auf das rohe 4-Isopropyl-3-methyl-phenol erfolgen, vorzugsweise mit 0,5 bis 10 Gewichtsäquivalenten.

Sofern gewaschen wird, kann das Waschen beispielsweise bei -10°C bis zum Siedepunkt des eingesetzten Lösungsmittels erfolgen.

Sofern Umkristallisiert wird, kann das Umkristallisieren beispielsweise mit 0,5 bis 20 Gewichtsäquivalenten des Lösungsmittels bezogen auf das rohe 4-Isopropyl-3-methyl-phenol erfolgen, vorzugsweise mit 0,5 bis 5 Gewichtsäquivalenten, wobei das Löslichkeitsverhalten in Abhängigkeit von der Temperatur Berücksichtigung finden kann und vorzugsweise auch findet.

Es ist für den Fachmann in einem oder wenigen Vorversuchen leicht möglich geeignete Bedingungen sowohl für das Waschen als auch für das Umkristallisieren zu identifizieren.

In einer Ausführungsform erfolgt das Umkristallisieren unter Hinzufügen von Filtrationshilfsmitteln oder Aktivkohle wobei dann vor Kristallisation des 4-Isopropyl-3-methyl-phenols aus dem Lösungsmittel das oder die Filtrationshilfsmittel bzw. die Aktivkohle durch Filtration wieder entfernt wird.

Schritt c) kann gegebenenfalls wiederholt werden. Sofern Schritt c) ein Umkristallisieren beinhaltet, ist die Wiederholung des Schrittes c) nicht bevorzugt.

Der besondere Vorteil der Erfindung ist darin zu sehen, dass 4-Isopropyl-3-methyl-phenol als Nebenkomponente bei der Thymol-Herstellung trotz Vorliegen sehr vieler weiterer, bei Raumtemperatur ebenfalls festen Nebenkomponenten, effizient und in außerordentlich hoher Reinheit gewonnen werden kann. Die Reinheit beträgt, sofern in Schritt c) gewaschen wird, typischerweise 98 bis 99 Gew.-%, beim Umkristallisieren typischerweise bei 98,5 bis 99,99 Gew.-%, vorzugsweise bei 99,8 bis 99,99 Gew.-%.

### Beispiel 1:

21,1 kg eines 60°C warmen, schwarzen Destillationsrückstandes aus der Herstellung von Thymol durch Umsetzung von m-Kresol mit Propen in Gegenwart eines Katalysators, der Destillationsrückstand enthaltend 21,5 Gew.-% Thymol, 7,0 Gew.-% 3-Isopropyl-5-methylphenol, 25,0 Gew.-% 4-Isopropyl-3-methyl-phenol, 21,5 Gew.-% 2,6-Diisopropyl-3-methyl-phenol, 21,0 Gew.-% 2,4-Diisopropyl-5-methyl-phenol sowie etwa 22 verschiedene in Summe ca. 4 Gew.-% sonstiger alkylierter Kresole wurden in einem 25-1 fassenden Reaktor innerhalb von 16 Stunden unter Rühren langsam auf Raumtemperatur abgekühlt, wobei das 4-Isopropyl-3-methyl-phenol auskristallisierte. Das ausgefallene, rohe 4-Isopropyl-3-methyl-phenol wurde abfiltriert. Es wurden 5,26 kg 4-Isopropyl-3-methyl-phenol mit einer Reinheit von 83,4 Gew.-% erhalten, was einer Ausbeute von 82,3 Gew.-% des im Destillationsrückstand vorhandenen 4-Isopropyl-3-methyl-phenol entsprach.

### Beispiel 2:

Je 50 g des rohen 4-Isopropyl-3-methyl-phenols gemäß Beispiel 1 wurden mit 150 g eines organischen Lösungsmittels bei 20°C unter Rühren auf einer Nutsche gewaschen. Die Ergebnisse sind in Tabelle 1 wiedergegeben.

**Tabelle 1:**

| **Beispiel** | **Lösungsmittel** | **Ausbeute [Gew.-%]** | **Reinheit [Gew.-%]** |
|---|---|---|---|
| 2a | Cyclohexan | 90 | 98,53 |
| 2b | n-Hexan | 94 | 98,08 |
| 2c | Dichlormethan | 63 | 98,76 |
| 2d | Chlorbenzol | 90 | 98,87 |
| 2e | Methylcyclohexan | 92 | 98,85 |
| 2f (zum Vergleich) | Ethylacetat | 0 | - |
| 2g (zum Vergleich) | tert.-Butylmethylether | 0 | - |
| 2h (zum Vergleich) | Isopropanol | 0 | - |
| 2i (zweimaliges Waschen) | Methylcyclohexan | 86 | 99,37 |

### Beispiel 2k:

Je 50 g des rohen 4-Isopropyl-3-methyl-phenols gemäß Beispiel 1 wurden mit 188 g Methylcyclohexan und 0,5 g Aktivkohle unter Rühren auf 85°C erwärmt und 15 min bei dieser Temperatur gerührt und die Aktivkohle abfiltriert. Das Filtrat wurde dann unter Rühren auf Raumtemperatur abkühlen gelassen. Das dabei auskristallisierte p-Thymol wurde abfiltriert und getrocknet. Man erhielt insgesamt 75 Gew.-% des eingesetzten p-Thymols als schneeweiße Nadeln mit einer Reinheit von 99,90 Gew.-%.

## Patentansprüche

1. Verfahren zur Herstellung von 4-Isopropyl-3-methyl-phenol aus Reaktionsmischungen, die bei der Umsetzung von meta-Kresol mit Propen in Gegenwart eines Katalysators erhalten werden, **dadurch gekennzeichnet, dass**
a) Thymol und nicht-umgesetztes meta-Kresol destillativ aus der Reaktionsmischung weitgehend abgetrennt werden wobei ein Destillationsrückstand gewonnen wird und
b) aus dem gemäß Schritt a) verbleibenden Destillationsrückstand rohes 4-Isopropyl-3-methyl-phenol durch Kristallisation gewonnen wird und
c) das gemäß Schritt b) erhaltene, rohe 4-Isopropyl-3-methyl-phenol mit einem organischen Lösungsmittel behandelt wird, das zu mindestens 80 Gew.-% aus Kohlenwasserstoffen oder halogenierten Kohlenwasserstoffen oder einer Mischung daraus besteht.

2. Verfahren nach Anspruch 1, **dadurch gekennzeichnet, dass** der Rückstand gemäß Schritt a) 10 bis 30 Gew.-% 4-Isopropyl-3-methyl-phenol enthält.

3. Verfahren nach Anspruch 1 oder 2, **dadurch gekennzeichnet, dass** in Schritt b) die Kristallisation durch Abkühlen des gemäß Schritt a) verbleibenden Rückstandes erfolgt, wobei die Temperaturdifferenz beim Abkühlen 30 K oder mehr vorzugsweise 40 K oder mehr, bevorzugt 40 bis 100 K beträgt.

4. Verfahren nach einem der Ansprüche 1 bis 3, **dadurch gekennzeichnet, dass** während des Abkühlens in Schritt b) gerührt wird.

5. Verfahren nach einem der Ansprüche 1 bis 4, **dadurch gekennzeichnet, dass** in Schritt b) die Kristallisation durch Animpfen mit kristallinem 4-Isopropyl-3-methyl-phenols induziert oder beschleunigt wird.

6. Verfahren nach einem der Ansprüche 1 bis 5, **dadurch gekennzeichnet, dass** der Reinheitsgrad des in Schritt b) erhaltenen, rohen 4-Isopropyl-3-methyl-phenols 75 bis 90 Gew.-%, vorzugsweise 80 bis 90 Gew.-% beträgt.

7. Verfahren nach einem der Ansprüche 1 bis 6, **dadurch gekennzeichnet, dass** der Anteil des in Schritt b) erhaltenen, rohen 4-Isopropyl-3-methyl-phenols 60 bis 90 Gew.-%, vorzugsweise 70 bis 90 Gew.-% bezogen auf den Gehalt an 4-Isopropyl-3-methyl-phenol in dem für Schritt b) eingesetzten Destillationsrückstand beträgt.

8. Verfahren nach einem der Ansprüche 1 bis 7, **dadurch gekennzeichnet, dass** das in Schritt c) benutzte organische Lösungsmittel zu mindestens 80 Gew.-%, vorzugsweise zu mindestens 90 Gew.-% und besonders bevorzugt zu mindestens 98 Gew.-% aus Kohlenwasserstoffen oder halogenierten Kohlenwasserstoffen oder einer Mischung daraus besteht, wobei besonders bevorzugt als Lösungsmittel ausschließlich Kohlenwasserstoffe oder halogenierte Kohlenwasserstoffe verwendet werden, wie sie in technischer oder Laborqualität kommerziell erhältlich sind.

9. Verfahren nach einem der Ansprüche 1 bis 8, **dadurch gekennzeichnet, dass** die Kohlenwasserstoffe oder halogenierte Kohlenwasserstoffe solche sind, die bei 1013 hPa einen Siedepunkt von 30 bis 160°C aufweisen.

10. Verfahren nach einem der Ansprüche 1 bis 9, **dadurch gekennzeichnet, dass** die Kohlenwasserstoffe Cyclopentan, Cyclohexan, n-Hexan, 2-Methylpentan, 3-Methylpentan, Methylcyclopentan, 2,2-Dimethylbutan, 2,3-Dimethylbutan, Methylcyclohexan, 2,2-Dimethylpentan, 2,3-Dimethylpentan, 2,4-Dimethylpentan, n-Heptan, n-Oktan, Isooctan oder Mischungen davon sind, wobei Methylcyclohexan besonders bevorzugt ist.

11. Verfahren nach einem der Ansprüche 1 bis 10, **dadurch gekennzeichnet, dass** die halogenierten Kohlenwasserstoffe Dichlormethan, Chlorbenzol, die isomeren Dichlorbenzole oder Mischungen der vorgenannten Lösungsmittel sind, wobei Dichlormethan und Chlorbenzol bevorzugt sind.

12. Verfahren nach einem der Ansprüche 1 bis 11, **dadurch gekennzeichnet, dass**, sofern gewaschen wird, das Waschen mit 0,5 bis 100 Gewichtsäquivalenten des Lösungsmittels bezogen auf das rohe 4-Isopropyl-3-methyl-phenol erfolgt, vorzugsweise mit 0,5 bis 10 Gewichtsäquivalenten.

13. Verfahren nach einem der Ansprüche 1 bis 11, **dadurch gekennzeichnet, dass**, sofern umkristallisiert wird, das Umkristallisieren mit 0,5 bis 20 Gewichtsäquivalenten des Lösungsmittels bezogen auf das rohe 4-Isopropyl-3-methyl-phenol erfolgt, vorzugsweise mit 0,5 bis 10 Gewichtsäquivalenten.

14. Verfahren nach einem der Ansprüche 1 bis 11 oder 13, **dadurch gekennzeichnet, dass** Schritt c) durch Umkristallisieren erfolgt und das Umkristallisieren unter Hinzufügen von Filtrationshilfsmitteln oder Aktivkohle erfolgt.

15. Verfahren nach einem der Ansprüche 1 bis 14, **dadurch gekennzeichnet, dass** Schritt c) wiederholt wird.
